# EUROPÄISCHE PATENTSCHRIFT

(11) **EP 0 727 226 B1**
(45) Veröffentlichungstag und Bekanntmachung des Hinweises auf die Patenterteilung: **09.04.2003**
(21) Anmeldenummer: 96100268.0
(22) Anmeldetag: 10.01.1996
(51) Int. Cl.: A61L 2/02, A61L 2/16

(54) **Entfernen von Viren durch Ultrafiltration aus Proteinlösungen**
Process for removing viruses from protein solutions by ultrafiltration
Procédé de suppression de virus dans des solutions de protéines par ultrafiltration

(30) Priorität: 09.02.1995 DE 19504211
(43) Veröffentlichungstag der Anmeldung: 21.08.1996
(73) Patentinhaber: Aventis Behring Gesellschaft mit beschränkter Haftung, 35002 Marburg (DE)
(72) Erfinder: Bernhardt, Dieter, Dr., D-35091 Cölbe (DE); Gröner, Albrecht, Dr., D-64342 Seeheim (DE); Nowak, Thomas, Dr., D-35460 Staufenberg-Mainzlar (DE)

(56) Entgegenhaltungen:
- EP-A- 0 302 949
- WO-A-90/15992
- WO-A-91/12027
- FR-A- 2 263 781

## Beschreibung

Die Erfindung betrifft die Entfernung von Viren aus wäßrigen Lösungen, in der Regel Proteinlösungen, durch Ultrafiltration. Die zu entfernenden Viren werden dabei durch Inkubation mit hochmolekularen, an diese bindenden Liganden, vorzugsweise spezifische Antikörper, vergrößert, so daß einerseits der Abtrenneffekt verbessert wird und andererseits ein jetzt wählbarer größerer Porendurchmesser der eingesetzten Filter noch die Abtrennung auch kleinerer Viren von in Proteinlösungen vorhandenen größeren Proteinmolekülen ermöglicht sowie gegebenenfalls die Filtrationsgeschwindigkeit erhöht wird.

Proteine, aus Humanplasma gereinigt und konzentriert, werden zur Therapie und Prophylaxe bei Erkrankungen des Menschen eingesetzt. Diese Produkte werden aus Plasmapools hergestellt, die aus ca. 10.000 Einzelspenden bestehen. Da einige dieser Spenden potentiell mit humanpathogenen Viren wie HIV-1/2, Hepatitis B-Virus, Hepatitis C-Virus und anderen Viren kontaminiert sein können, besteht die Möglichkeit einer durch die Applikation der Plasmaproteine verursachten Infektion. Um diese Kontaminationsgefahr zu minimieren, werden nur Spenden gesunder Spender gewonnen, die zusätzlich auf Infektionsmarker (Antikörper gegen HIV 1 und HIV 2, HBsAg, Antikörper gegen HCV und erhöhte Leberwerte (ALT)) untersucht werden; positive Spenden werden aussortiert und nicht zur Gewinnung von Plasmaproteinen eingesetzt. Die bei der industriellen Herstellung der Plasmaprotine eingesetzten Reinigungs- und Konzentrierungsschritte sowie insbesondere gezielt in die Produktion eingeführte Schritte zur Viruseliminierung und/oder -inaktivierung führen zu Plasmaproteinen mit einem sehr hohen Sicherheitsstandard.

Um die Sicherheit der Plasmaproteine noch weiter zu erhöhen, wurde der Einsatz von Filtrationsverfahren, z.B. Dead-End- und Tangentialflußfiltration untersucht, um evtl. vorhandene Viren aus der Proteinlösung zu eliminieren. Filtermodule zur Elimination von Viren werden von verschiedenen Firmen hergestellt (DiLeo, A.J. et al. Biologicals 21, 275-286 (1993); DiLeo A.J. et al. Biologicals 21, 287-296 (1993); Burnout, T. et al., Vox Sang. 67, 132-138 (1994)). So stellt z.B. die Firma Asahi Chemical Industry Co., LTD, Tokyo, Japan, Filtermodule unter Angabe einer definierten (durchschnittlichen) Porengröße her, während z.B. die Firma Millipore Corp., Bedford, MA, USA Filtermodule mit Angabe einer nominellen Molekulargewichts-Trenngrenze herstellt.

Bei unseren Untersuchungen hat sich herausgestellt, daß Viren in Abhängigkeit von ihrem Durchmesser (HIV: 80-100 nm; HCV: 40-60 nm; HBV: 40-45 nm; Picornaviren: 24-30 nm; Parvoviren: 18-25 nm) von Filtern mit verschiedenen Porengrößen mit unterschiedlichen Raten zurückgehalten werden: (I) Filter mit einem mittleren Porendurchmesser von 75 nm halten HIV im wesentlichen zurück, während die anderen genannten Viren in das Filtrat gelangen; (II) Filter mit einem mittleren Porendurchmesser von 35 nm halten HIV vollständig und HCV und HBV zu einem hohen Anteil zurück, während z.B. Picornaviren und Parvoviren in das Filtrat gelangen; (III) Filter mit einem mittleren Porendurchmesser von 15 nm halten HIV, HCV und HBV zurück, sowie zu einem hohen Anteil z.B. Picornaviren und Parvoviren. Die Filtration durch einen Filter mit einem mittleren Porendurchmesser von 15 nm führt zu einer generellen Erhöhung der Virussicherheit von Plasmaproteinen. Da die meisten Plasmaproteine jedoch ein so hohes Molekulargewicht besitzen, daß sie nicht durch einen 15 nm-Filter filtrierbar sind, d.h. ebenfalls zurückgehalten werden, sind für die Filtration der meisten Plasmaproteine nur Filter mit einem mittleren Porendurchmesser von 35 nm (bzw. einer nominellen Molekulargewichts-Trenngrenze von 70.000 D bis 100.000 D) geeignet, die jedoch zumindest Picornaviren (wie z.B. Hepatitis A Virus) und Parvoviren (wie das humanpathogene Parvovirus B 19) nicht in ausreichendem Maße aus Plasmaproteinen entfernen.

Es bestand daher die Aufgabe, auch bei kleinen Viren durch Filtration eine ausreichende, d.h. vollständige Rückhaltung zu erreichen, sowie Filtrationsverfahren auch auf solche Proteine anwendbar zu machen, die schon von ihrer Größe einem kleinen Virus nahekommen. Zusätzlich sollte die Filtrationsgeschwindigkeit möglichst gesteigert werden.

Durch die vorliegende Erfindung wird die Aufgabe dadurch gelöst, daß die zu entfernenden Viren durch Bindung an in Lösung befindlichen Antikörper, besonders bevorzugt monoklonale Antikörper, prinzipiell aller Subklassen aber vorzugsweise Subklasse IgG oder IgM oder noch bindungsfähige Teile derselben, die gegebenenfalls gentechnisch modifiziert oder vergrößert sind, so vergrößert werden, daß sie durch Filtration zurückhaltbar sind. Es ist mit dieser Methode sogar möglich, relativ große Proteine wie Faktor VIII bzw. von Willebrandfaktor von solchen "vergrößerten" Viren durch Filtration zu trennen, wobei die Porengröße jetzt so gewählt werden kann, daß die Proteine passieren und die "vergrößerten" Viren zurückgehalten werden. Überdies kann durch Wahl einer nun möglichen größeren Porenweite die Filtrationsgeschwindigkeit erhöht werden. In der allgemeinsten Form sind durch die vorliegende Erfindung beliebige Bestandteile einer wässrigen Lösung sofern durch Bindung an hochmolekulare Liganden vergrößerbar, von den dann kleineren Bestandteilen über einen Filtrationsschritt abtrennbar.

Humanpathogene Viren, wie z.B. HBV, HCV, HIV, Picornaviren und Parvoviren, können trotz Spenderauswahl in einem Plasmapool vorhanden sein. Diese Viren binden an in der Proteinlösung vorhandene Antikörper und zwar entweder an im

Plasmapool vorliegende Antikörper während einer Inkubation des kryoarmen Plasmas bzw. des resuspendierten Kryopräzipitates bei 2°C bis 37°C von 15 Minuten bis 36 Stunden, vorzugsweise bei 2°C bis 8°C über einen Zeitraum von 2 bis 36 Stunden, insbesondere von 4 bis 18 Stunden bzw. bei 10°C bis 25°C über einen Zeitraum von 15 Minuten bis 18 Stunden; vorzugsweise von 30 Minuten bis 6 Stunden oder an gezielt kurz vor der Filtration zu der zu filtrierenden Plasmaproteinlösung zugegebene Antikörper, die in nativer oder chemisch bzw gentechnisch modifizierter Form eingesetzt werden können (z.B. kryoarmes Plasma, Immunglobulinfraktionen, gereinigte Immunglobuline menschlichen oder tierischen Ursprungs, monoklonale Antikörper) während einer Inkubation der Proteinlösung bei 2°C bis 37°C von 15 Minuten bis 36 Stunden, vorzugsweise bei 2°C bis 8°C über einen Zeitraum von 2 bis 36 Stunden, insbesondere von 4 bis 18 Stunden bzw. bei 10°C bis 30°C über einen Zeitraum von 15 Minuten bis 8 Stunden, vorzugsweise von 30 Minuten bis 4 Stünden. Die so gebildeten Virus-Antikörperkomplexe lassen sich durch Filtration, z.B. Dead-End Filtration oder vorzugsweise Tangentialflußfiltration aus der Plasmaproteinlösung entfernen.

### Beispiel 1:

Bovines Parvovirus (BPV; ATCC VR-767), als Modellvirus für das humanpathogene Parvovirus B 19, wurde in diploiden fötalen Rinderlungenzellen in EME-Medium mit 5 % FKS vermehrt und danach durch niedertourige Zentrifugation (2.000 g, 15 Minuten, 4°C) von Zellen und Zellresten getrennt; der virushaltige Überstand wurde aliquotiert und bis zur Untersuchung bei -70°C gelagert. Zum Vergleich wurde Porcines Parvovirius (PPV; ATCC VR-742) untersucht; PPV wurde wie BPV vermehrt und gewonnen, jedoch in einer permanenten Schweinenieren-Zellinie (IB-RS-2 D10; ATCC CRL 1835).

Folgende Versuchsansätze wurden gemischt, 1 Stundo boi 20°C inkubiert und nachfolgend durch BMM Process Filter PLANOVA™ 35 (Fa. Asahi Chemical Industry Co., LTD., Tokyo, Japan) entsprechend den Angaben der Herstellerfirma filtriert. Der Infektiositätstiter (CCID₅₀: Zellkulturinfektiöse Dosis 50 %) wurde im Ausgangsmaterial und nach der Filtration im Filtrat bestimmt

| | **Versuch A** | ***Versuch B*** | **Versuch C** | **Versuch D** | **Versuch E** |
|---|---|---|---|---|---|
| Protein-lösung (260 ml) | Albumin (5%) | ***Albumin* (5%)** | Albumin (5%) | Albumin (5 %) | . Albumin (5 %) |
| Virus-material (30 ml) | BPV | ***BPV*** | BPV | PPV | PPV |
| Antikörper-haltige Lösung (30 ml) | PBS (keine Antikörper) | ***Human- serum (B19- positiv*** /***ELISA)*** | Human-serum (B19-negativ / ELISA) | Human-serum (B19-positiv / ELISA) | Human-serum (B19-negativ / ELISA) |
| Infektionstiter vor Filtration (CCID₅₀) | 10^{5,2} | ***10***^{***4,2***} | 10,^{5,1} | 10^{6,4} | 10^{6,2} |
| Infektionstiter nach Filtration (CCID₅₀) | 10,^{4,9} | ***<10***^{***0,5***} | 10^{4,3} | 10^{6,0} | 10^{5,9} |

Da BPV mit B19 serologisch kreuzreagiert (Bernhardt, D. et al., Tierärztl. Umschau 49, 481-483 (1994), binden Antikörper gegen B19 aus humanem Plasma auch an BPV, jedoch nicht an PPV. Die während der Inkubation entstandenden Antigen-Antikörper-Komplexe sind so groß, daß sie im Gegensatz zu nicht komplexiertem Antigen nicht filtrierbar sind.

### Beispiel 2:

Ein zugelassener Poliovirus-Impfstoff zur oralen Impfung (Oral-Virelon®; lebend attenuierter Impfstoff) wurde in einer Proteinlösung aus 10 % fötalem Kälberserum in DMEM suspendiert; zu einem Teil dieser Virussuspension wurde gereinigtes Immunglobulin (Beriglobin®) gegeben, der andere Teil wurde mit demselben Volumen PBS versetzt. Nach einer Inkubation von 2 Stunden bei 15°C wurden die Proben filtriert (Sartocon®-Micro, 100.000 D nominelle Molekulargewichtstrenngrenze). Da die Immunglobuline Polioviren neutralisieren, so daß ein Infektiositätstest bei diesem Versuchsglied keine Aussage über eine Abreicherung durch die Filtration erlaubt, wurden Viren in Proben der Retentate und der Filtrate nach einem pH-Shift (pH·4,10 Minuten; dann Zentrifugation durch ein Saccharosekissen von 25 % (w/w) Saccharose bei 20.000 g, 45 Minuten, 4°C und Resuspension des Pellets in PBS pH 7,2) in einem Dot-Blot auf Nitrocellulose nachgewiesen. Die resuspendierten Proben wurden 1:2 in Tris/Glycin-Puffer pH 8,3 vorverdünnt und nachfolgend in einer 1:3-Verdünnungsreihe weiter verdünnt; je 100 µl jeder Verdünnung wurde auf Nitrozellulosefilter (0,45 um Porengröße) aufgebracht, die Membran mit Magermilchpulver (3 %) geblockt, mit Antiserum gegen Polioviren (vom Kaninchen) 1 Stunde bei 37°C inkubiert und danach mit POD-markierten Anti-Kaninchen-Antikörpern weiter inkubirt. Die gebundenen Antikörper wurden mit 4-Chloro-1-Naphtol/H₂O₂ sichtbar gemacht (Durchführung des Dot-Blots entsprechend Cardosa, M.J. & Tio, P.H., Bull. WHO, 69, 741-745, 1991).

| | ***Versuch A*** | **Versuch B** |
|---|---|---|
| Proteinlösung (260 ml) | ***10 % FKS in DMEM*** | 10 % FKS in DMEM |
| Virusmaterial (30 ml) | ***Poliovirus*** | Poliovirus |
| Antikörper-haltige Lösung (30 ml) | ***gereinigte Immunglobuline (Beriglobin)*** | PBS |
| Titer im Dot Blot vor Filtration | ***1024*** | 1024 |
| Titer im Dot Blot nach Filtration | ***<2*** | 256 |

Die Poliovirus-Antikörperkomplexe sind im Gegensatz zu nicht komplexierten Antigenen nicht filtrierbar; eine Entfernung aus der Proteinlösung ist deshalb duch die Zugabe von Immunglobulinen erzielbar.

## Patentansprüche

1. Verfahren zur Entfernung von Viren aus Lösungen therapeutischer Plasmaproteine, **dadurch gekennzeichnet, dass** die zu entfernenden Viren
a) zunächst durch Bindung an in Lösung befindlichen Antikörpern vergrößert werden und dann
b) durch Filtration durch Hohlfaser- oder Membranfilter definierter Porengröße von den therapeutischen Plasmaproteinen getrennt werden.

2. Verfahren nach Anspruch 1, **dadurch gekennzeichnet, dass** der Antikörper ein chemisch oder gentechnisch modifizierter Antikörper ist.

3. Verfahren nach Anspruch 2, **dadurch gekennzeichnet, dass** der Antikörper ein monoklonaler Antikörper, oder ein noch an Viren bindender Teil davon ist.

4. Verfahren nach einem der Ansprüche 1 bis 3, **dadurch gekennzeichnet, dass** die Filtration im Tangentialflussverfahren durchgeführt wird.

## Claims

1. A method for removing viruses from protein solutions of therapeutic plasma proteins, which comprises the viruses to be removed
a) initially being increased in size by binding to antibodies present in solution, and then
b) being separated from the therapeutic plasma proteins by filtration through hollow fibre filters or membrane filters of defined pore size.

2. The method as claimed in claim 1, wherein the antibody is an antibody which is modified chemically or by genetic engineering.

3. The method as claimed in claim 2, wherein the antibody is a monoclonal antibody, or a part thereof which still binds to viruses.

4. The method as claimed in any of claims 1 to 3, wherein the filtration is carried out via the tangential flow method.

## Revendications

1. Procédé pour l'élimination de virus de solutions de protéines plasmatiques thérapeutiques, **caractérisé en ce que** les virus à éliminer
a) sont d'abord agrandis par liaison à des anticorps présents en solution et ensuite
b) séparés des protéines plasmatiques thérapeutiques par filtration à travers des filtres à membrane ou à fibres creuses, de taille de pore définie.

2. Procédé selon la revendication 1, **caractérisé en ce que** l'anticorps est un anticorps modifié chimiquement ou génétiquement.

3. Procédé selon la revendication 2, **caractérisé en ce que** l'anticorps est un anticorps monoclonal ou une partie de celui-ci se liant aux virus.

4. Procédé selon l'une quelconque des revendications 1 à 3, **caractérisé en ce que** la filtration est effectuée par le procédé de flux tangentiel
